# EUROPEAN PATENT SPECIFICATION

(11) **EP 1 349 825 B1**
(45) Date of publication and mention of the grant of the patent: **19.10.2005**
(21) Application number: 01952202.8
(22) Date of filing: 21.06.2001
(51) Int. Cl.: C07C 51/265, C07C 63/26, C07C 51/21, C07C 67/39

(54) **IMPROVED PROCESS FOR PRODUCING CARBOXYLIC ACIDS**
VERBESSERTES VERFAHREN ZUR HERSTELLUNG VON CARBONSÄUREN
PROCEDE AMELIORE DE PRODUCTION D'ACIDES CARBOXYLIQUES

(30) Priority: 10.01.2001 US 757458; 11.01.2001 WO PCT/US01/00825; 19.06.2001 US 884381
(43) Date of publication of application: 08.10.2003
(73) Proprietor: E.I. DU PONT DE NEMOURS AND COMPANY, Wilmington, Delaware 19898 (US)
(72) Inventor: TURNER, John, Arthur, Stokesley, Yorkshire TS9 5AS (GB); HOUSLEY, Samuel, Duncan, Cleveland TS15 0JG (GB)
(74) Representative: Cockerton, Bruce Roger
(86) International application number: PCT/US2001/020109
(87) International publication number: WO 2002/055468

(56) References cited:
- EP-A- 0 686 633
- WO-A-98/07677
- US-A- 3 595 908
- US-A- 4 215 052
- US-A- 4 342 876

## Description

The present invention relates to an improved process for producing carboxylic acids or their esters by catalytic liquid phase oxidation of a corresponding precursor in a suitable solvent. More particularly, the present invention is an improved process for the catalytic liquid phase oxidation of paraxylene to produce terephthalic acid which comprises sequential steps of feeding the reactants to a first reaction zone at elevated pressure wherein the temperature and the uptake of oxygen are controlled and any terephthalic acid which forms remains in solution, and then feeding the resulting reaction medium to a second reaction zone to complete the oxidation reaction.

Practically all terephthalic acid is produced on a commercial scale by catalytic, liquid phase air oxidation of paraxylene. Commercial processes use acetic acid as a solvent and a multivalent heavy metal or metals as catalyst. Cobalt and manganese are the most widely used heavy metal catalysts, and bromine is used as a renewable source of free radicals in the process.

Acetic acid, air (molecular oxygen), paraxylene and catalyst are fed continuously into an oxidation reactor that is maintained at from 175°C to 225°C and 1000-3000 kPa (i.e., 10-30 atm). The feed acetic acid:paraxylene ratio is typically less than 5:1. Air is added in amounts in excess of the stoichiometric requirements for full conversion of the paraxylene to terephthalic acid, to minimize formation of undesirable by-products, such as color formers. The oxidation reaction is exothermic, and heat is removed by allowing the acetic acid solvent to vaporize. The corresponding vapor is condensed and most of the condensate is refluxed to the reactor, with some condensate being withdrawn to control reactor water concentration (two moles of water are formed per mole of paraxylene reacted). The residence time is typically 30 minutes to 2 hours, depending on the process. Depending on oxidation reactor operating conditions, e.g., temperature, catalyst concentration and residence time, significant degradation of the solvent and precursor can occur, which, in turn, can increase the cost of operating the process.

The effluent, i.e., reaction product, from the oxidation reactor is a slurry of crude terephthalic acid (TA) crystals which are recovered from the slurry by filtration, washed, dried and conveyed to storage. They are thereafter fed to a separate purification step or directly to a polymerization process. The main impurity in the crude TA is 4-carboxybenzaldehyde (4-CBA), which is incompletely oxidized paraxylene, although p-tolualdehyde and p-toluic acid can also be present along with undesirable color formers. By conducting the oxidation reaction according to the invention as described in greater detail below, it is possible to substantially reduce the formation of impurities in the final TA product and effectively control solvent and precursor degradation.

### SUMMARY OF THE INVENTION

The present invention is an improved process for catalytic liquid phase oxidation of paraxylene to produce terephthalic acid which comprises sequential steps of feeding the reactants, including a suitable solvent, to a first reaction zone at elevated pressure wherein the temperature and the uptake of oxygen are controlled and any terephthalic acid which forms remains in solution, and then feeding the resulting reaction medium to a second oxidation reaction zone. The process comprises:
(a) forming a feed stream comprising solvent and oxidation catalyst at a pressure in the range of from at least about 2,000 kPa up to 10,000 or higher;
(b) continuously and simultaneously feeding (1) the feed stream, (2) paraxylene and (3) a supply of oxygen to a first reaction zone to form a reaction medium in which the solvent:paraxylene mass ratio is in the range of from 10-25:1;
(c) limiting the uptake of oxygen within the reaction medium in the first reaction zone to a value which is less than 50% of the oxygen required for full conversion of the paraxylene to terephthalic acid; and
(d) feeding the reaction medium to a second reaction zone while simultaneously reducing the pressure of the reaction medium to a value in the range of from 1,200 kPa to 2,000 k.Pa.

The resulting terephthalic acid can be recovered from the reaction medium exiting the second reaction zone by any convenient means.

While the invention is described herein in terms of an improved oxidation system for converting paraxylene to terephthalic acid, it will be recognized that the invention is applicable to producing a range of carboxylic acids or their esters, and particularly phthalic acids or their esters, by catalytic liquid phase oxidation of a corresponding precursor in a suitable solvent. The invention resides in the discovery that the conversion of the precursor to its corresponding carboxylic acid can be substantially improved by carrying out the oxidation reaction in at least two stages, or zones, which comprise:
(1) forming a feed stream comprising solvent and oxidation catalyst at an elevated pressure of at least about 2,000 kPa, and then continuously and simultaneously feeding the feed stream, the precursor and a supply of oxygen to a high pressure first reaction zone to form a reaction medium in which the solvent:precursor mass ratio is also relatively high, i.e., in the range of from 10-25:1, although the preferred ratio for economy and operability is from 10-20:1; and
(2) feeding the reaction medium from the first reaction zone to a second reaction zone, where the oxidation reaction runs to completion.

In addition to maintaining the solvent:precursor mass ratio as described, the uptake of oxygen in the first reaction zone is limited to a value which is less than 50% of the oxygen required for full conversion of the precursor to its corresponding carboxylic acid or ester, which can have one, two, three or more acid groups, depending on the precursor. Oxygen uptake in the first reaction zone is controlled by one or more of the following methods: (i) maintaining oxygen supply (i.e., oxygen concentration) within a predetermined range; (ii) maintaining catalyst concentration within a predetermined range; (iii) limiting the residence time (defined as the reactor liquid volume divided by the reactor feed rate) within the first reaction zone to less than about 6 minutes, but preferably less than 4 minutes; and (iv) optionally removing heat from the reaction zone to maintain the temperature of the reaction medium as it exits the first reaction zone at a value which is below about 210°C.

Simultaneously while feeding the reaction medium to the second reactor the pressure of the reaction medium is reduced to a value in the range of from 1,200 kPa to 2,000 kPa. The carboxylic acid which results can be recovered from the final reaction medium, which is typically a slurry of acid crystals, by conventional methods.

According to a preferred embodiment of the invention, oxygen is dissolved directly into the feed stream comprising solvent and oxidation catalyst, and the oxygenated feed stream is then fed continuously and simultaneously with the precursor into the first oxidation reaction zone, which is a plug flow reaction zone. Immediately upon entering the first reaction zone the precursor, e.g., paraxylene, is thoroughly mixed with the oxygenated solvent to initiate the reaction. By controlling the oxygen supply, catalyst concentration, residence time in and/or temperature of the first reaction zone, it is possible to control, i.e., limit, the uptake of oxygen within the reaction medium to a value which is less than 50% of the oxygen required for full conversion of the precursor to its corresponding carboxylic acid. The reaction medium is then fed to a second, more conventional, reactor as described above.

The process of the invention is particularly applicable to producing terephthalic acid by catalytic liquid phase oxidation of paraxylene in a solvent comprising acetic acid and water.

### BRIEF DESCRIPTION OF THE DRAWINGS

Fig. 1 is a simplified schematic diagram of the process of the invention according to one embodiment.
Fig. 2 is a simplified schematic diagram of the process of the invention according to a preferred embodiment.
Fig. 3 is a simplified schematic diagram of an alternative to the process diagram shown in Fig. 2 wherein a back-mixed reactor is illustrated.

### DETAILED DESCRIPTION OF THE INVENTION

The present invention resides in the discovery that it is possible, when carrying out liquid phase catalytic oxidation of paraxylene in the presence of an acetic acid solvent, to effectively stage the oxidation reaction on a commercial scale into a first high pressure reaction zone followed by a second, more conventional, reaction zone and thereby substantially improve process efficiency and product quality.

The first reaction zone of the process is characterized by a relatively high solvent:precursor mass ratio in the range of from 10-25:1, or even higher as described in further detail according to one embodiment below, and a relatively high pressure, e.g., in the range of from at least 2,000 kPa up to 10,000 kPa or even higher. Higher solvent:precursor mass ratios may be used, for example, up to 30:1 or even higher, but best results for the overall process, when paraxylene is fed to the process exclusively through or via the first reaction zone, have been observed when the solvent:precursor mass ratio in the first reaction zone is in the range of from 10-20:1. The first reaction zone is optionally cooled to control the temperature of the reaction medium as it exits the first reaction zone at a value which is below about 210°C. Control of temperature, catalyst concentration, reactor residence time, and/or maintaining the oxygen supply to the first reaction zone within a predetermined range makes it possible to conveniently limit the uptake of oxygen within the reaction medium to a value which is less than 50% of the oxygen required for full conversion of the paraxylene to terephthalic acid.

Temperature control can be established, for example, by placing an internal cooling coil or other cooling device within the first reaction zone, by employing a cooling jacket to surround the reactor or by circulating the reaction medium through a heat exchanger located externally from the reactor.

Catalyst control can be established by, for example, routing some of the catalyst-containing mother liquor directly to the second reaction zone, bypassing the first reaction zone.

It is critical to avoid precipitation of solid TA onto any cooling surfaces in the first reaction zone. TA formation is limited by limiting oxygen uptake, and TA precipitation is also prevented by maintaining a high solvent:precursor ratio within the reaction medium, and by selecting an appropriate coolant (e.g., boiling water) and cooling means that avoids cold spots from forming at any location within the reaction medium.

On exiting the first reaction zone, the pressure of the reaction medium is reduced simultaneously as it is fed to a more conventional oxidation reactor. This reactor could typically be a stirred tank reactor or a bubble column reactor, for example. Pressure reduction can be conveniently accomplished by passing the reaction medium through one or a plurality of pressure letdown valves positioned about the periphery of the reactor. Best results have been obtained when the reaction medium is dispersed rapidly upon entering the second reactor. Rapid dispersion can be achieved by using established methods for dispersing paraxylene-containing feeds in conventional reactors. In a stirred tank reactor, for example, this would include injecting the reaction medium into the reactor below the liquid line in close proximity to the discharge from an agitator impeller. Rapid dispersion of the reaction medium can be achieved in a bubble column reactor by injecting the reaction medium in close proximity to the air feeds.

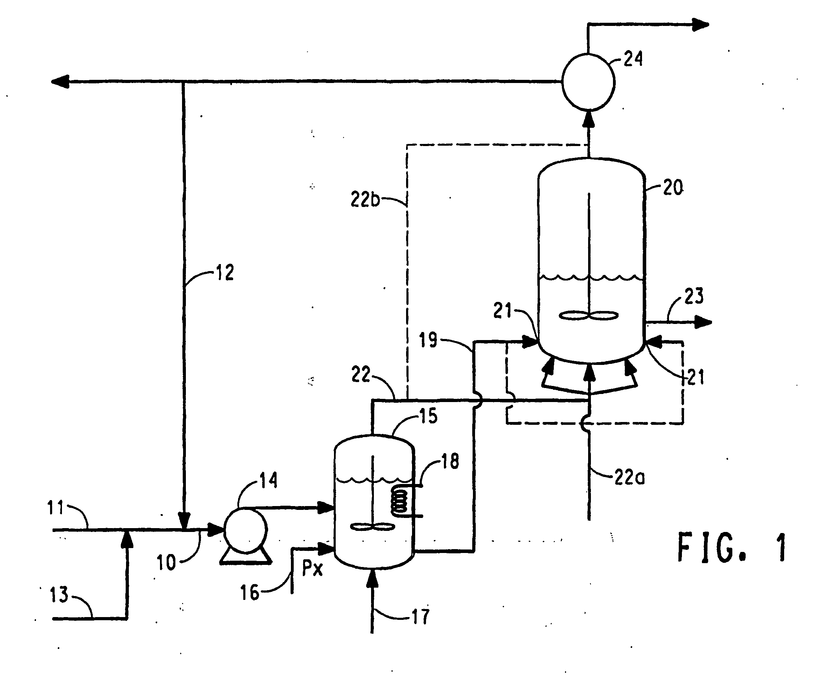

Referring now to the drawings, Fig. 1 is a simplified schematic diagram of the process of the invention according to one embodiment. As mentioned above, the process will be described as it relates to the production of terephthalic acid, although the invention is applicable to the production of a range of carboxylic acids or their esters and mixtures thereof.

The process is carried out by first forming a feed stream 10 comprising solvent, i.e., acetic acid and water, and oxidation catalyst. In practice the feed stream will comprise a mixture comprising (i) recycled solvent, recycled mother liquor and catalyst, line 11, (ii) reactor condensate from the second reactor, line 12, and (iii) fresh acetic acid make-up, line 13. The mixed feed stream will contain typical catalyst components (e.g., Co, Mn, Br), at generally diluted concentrations from what would normally be present when using a single conventional oxidation reactor. Optionally, but not shown, control of catalyst concentration in the first reaction zone can be achieved by bypassing some of the catalyst-containing mother liquor, line 11, directly to second reactor 20.

The mixed feed stream will generally have a temperature in the range of from 130°C to 160°C, based on the temperature of the various components which form the feed stream. However, the temperature of the feed stream is not critical.

The pressure of feed stream 10 is raised via a suitable pump 14 to a value of at least about, but generally in excess of, 2,000 kPa, and the feed stream is introduced continuously and simultaneously into a first stirred tank reactor 15 with paraxylene, via line 16, and a source of oxygen, via line 17.

The supply of oxygen via line 17 can be air, oxygen-enriched air, oxygen mixed with inert gas, such as, for example, carbon dioxide, or essentially pure oxygen. When the source of oxygen includes nitrogen or another inert carrier gas, the extent of cooling in the first reaction zone and its operating pressure are preferably chosen such that the vapor present in the first reaction zone is fuel-lean, i.e., the hydrocarbon content of the vapor is below the Lower Explosive Limit (LEL). When essentially pure oxygen is used as the source of oxygen, the extent of cooling in the first reaction zone and its operating pressure are preferably chosen such that there is no vapor phase present in the first reaction zone. Optionally, but not shown in Fig. 1, some oxygen can be pre-dissolved directly into feed 10 via a mixing device located downstream of the feed pump.

The paraxylene feed 16 may optionally be pre-mixed with acetic acid solvent and introduced into the system either upstream or downstream of feed pump 14. Optionally, but not shown, a portion of paraxylene feed 16 may bypass reactor 15 and be fed directly to second reactor 20. The reaction medium which results in the first reactor, without bypassing any paraxylene to the second reaction zone, has an acetic acid:paraxylene ratio in the range of from 10-25:1. Best results have been observed for this embodiment when the acetic acid:paraxylene mass ratio is from 10-20:1.

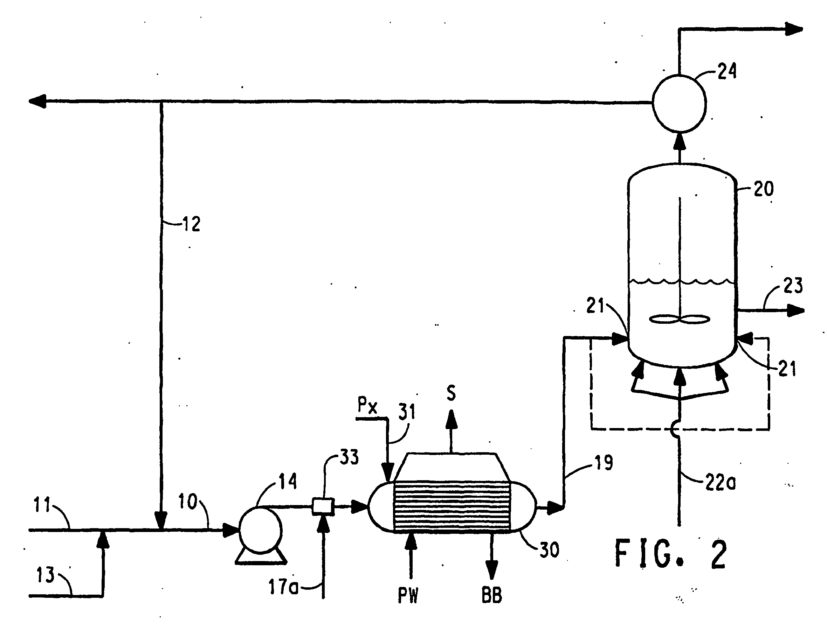

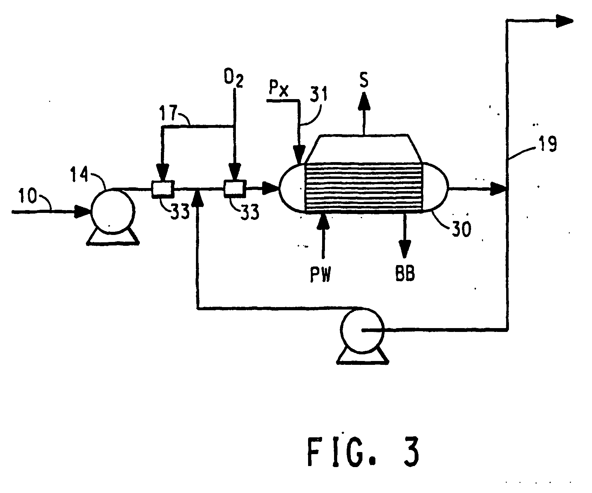

In cases where a portion of the paraxylene feed, i.e., line 16 in Fig. 1 and line 31 in Figs. 2 & 3, is arranged to bypass the first reactor and is fed directly to second reactor 20, the resulting solvent:paraxylene mass ratio in the reaction medium in the first reactor will adjust upward in response to that portion of the paraxylene which bypasses the first reactor, and the resulting mass ratio may reach a value in the range of up to 100:1 The paraxylene feed, line 16, should be dispersed rapidly upon entering the first reactor. This can be accomplished by using any of the established methods for rapidly dispersing paraxylene-containing feeds in conventional reactors. In a stirred tank reactor 15, as shown in the embodiment of the invention illustrated in Fig. 1, this would include injecting the feed in close proximity to the discharge from an agitator impeller. Although a stirred tank reactor is shown in Fig. 1, other conventional oxidation reactor configurations may also be used with satisfactory results.

The process is carried out in the presence of an oxidation catalyst system, which can be homogeneous or heterogeneous. A homogeneous catalyst is normally used and is selected from one or more heavy metal compounds, such as, for example, cobalt, manganese and/or zirconium compounds. In addition, the catalyst will normally also include an oxidation promoter such as bromine. The catalyst metals and oxidation promoter largely remain in solution throughout the process and are recovered and recycled, following product recovery, with fresh catalyst make-up as a solution.

The feed stream to the first reaction zone, line 10, contains typical oxidation catalyst components (e.g., Co, Mn, Br), but diluted by a factor of about 3 to 5 relative to the catalyst concentration in recycled mother liquor from product recovery, line 11. The catalyst concentration is subsequently raised to more conventional catalyst concentration levels when and as solvent is vaporized and removed overhead in the second reaction zone 20. The total catalyst metals concentration in the first reaction zone will typically lie in the range 150 to 1,000 ppm w/w, whereas the catalyst metals concentration in the second reaction zone will typically lie in the range 500 to 3,000 ppm w/w. When using a Co and Mn metal catalyst system, the total catalyst metals concentration in the first reaction zone should preferably be controlled at greater that about 250 ppm w/w for good catalyst selectivity and activity.

The oxidation reaction is highly exothermic. Depending on the oxygen uptake and solvent ratio and without a means of cooling the reaction, the heat of reaction could raise the temperature of the first reaction medium to a value higher than the second reactor operating temperature and/or higher than 210°C. A relatively low first reactor exit temperature is desirable to minimize solvent and precursor degredation (i.e., burn) and to eliminate solvent flashing as the: pressure of the reaction medium is reduced on entry into the second reaction zone. The first reaction zone may therefore include a cooling coil 18 or employ some other internal or external means for removing heat from the reactor (and reaction medium) to control the exit temperature of the reaction medium below 210°C, and preferably below the second reactor operating temperature. It is important that the temperature of the coolant is about 120°C or higher to prevent cold spots from forming and resulting in localized precipitation of terephthalic acid (TA).

Maintaining the supply of oxygen to the first reaction zone within a predetermined range and controlling the exit temperature, catalyst concentration and residence time of the reaction medium makes it possible to limit the uptake of oxygen within the reaction medium to a value which is less than 50% of the oxygen required for full conversion of the paraxylene to TA. Thus, according to the invention, paraxylene is converted in first reactor 15 primarily to TA intermediates, such as p-tolualdehyde, p-toluic acid and 4-CBA. Under the described process conditions, with effective exit temperature control, the first reactor will not produce any solid TA.

The reaction medium exiting first reactor 15 is fed via line 19 to a second reactor, i.e., oxidation zone, 20, which, as shown, can be a conventional, continuously stirred tank reactor. Simultaneously, the pressure of the reaction medium is reduced to a value in the range of from 1,200 kPa to 2,000 kPa. As described above, pressure reduction can be conveniently accomplished by passing the reaction medium through one or a plurality of pressure letdown valves or nozzles 21 positioned about the periphery of reactor 20 whereby the reaction medium is dispersed rapidly by injection into an agitator impeller region below the liquid line of the reactor. Process conditions within reactor 20, i.e., temperature, pressure, catalyst concentration and residence time, are within conventional ranges, although oxygen uptake is reduced for reduced oxidation intensity.

Where the source of oxygen to the first reactor includes nitrogen or another inert carrier gas, spent or excess air from first reactor 15, line 22, is mixed with a fresh supply of air or oxygen-containing gas, line 22a, and the resulting mixed feed gas stream is introduced and rapidly dispersed into the reaction medium in second reactor 20 by any convenient means. Alternatively, spent or excess air from first reactor 15 can be fed directly to condenser 24 as shown via dotted line 22b, with exclusively fresh air or oxygen-containing gas being fed to second reactor 20.

TA will precipitate to form a slurry within reactor 20, and it can be recovered from the reactor system via line 23 using conventional methods. Reactor overhead vapor from reactor 20, which will necessarily contain some acetic acid and water, is condensed via condenser 24, and most of the condensate is returned, i.e., recycled, via line 12 for feed stream make-up to first reactor 15. A proportion of the acetic acid and water condensate stream (so-called water draw off) is diverted to a solvent dehydration system to remove the water of reaction. Optionally, but not shown, a portion of the condensate may be returned to reactor 20, to the reactor headspace, via a reflux slinger, and/or to the reaction zone, via a separate feed line or by mixing with the existing feed stream, line 19.

Fig. 2 is a simplified schematic diagram of a preferred embodiment of the invention. The first reaction zone, i.e., first reactor 30, according to this embodiment is a plug flow reactor. The term "plug flow reactor" is used herein to define a generally elongated, or tubular, reaction zone in which rapid and thorough radial mixing of the reactants occurs as they flow through the tube or conduit. The invention, however, is intended to embrace any reactor configuration which approximates to a plug flow reaction zone.

As described above in connection with Fig. 1, feed stream 10 is a mixed feed stream comprising
(i) recycled solvent, recycled mother liquor and catalyst, via line 11, (ii) second reactor condensate, via line 12, and (iii) fresh acetic acid make-up, via line 13. Optionally, but not shown, control of catalyst concentration in the first reaction zone can be achieved by bypassing some of the catalyst-containing mother liquor, line 11, directly to second reactor 20. The supply of oxygen in this embodiment, line 17a, is essentially pure gaseous oxygen.

The mixed feed stream will generally have a temperature in the range of from 130°C to 160°C, depending on the temperature of the make-up streams. A temperature in the range of about 140°C has been found to be suitable for initiating the oxidation reactions.

The pressure of mixed feed 10 is raised to a value in the range of at least, but generally in excess of, 2,000 kPa by any suitable pumping means 14. The pressure is chosen to ensure that all of the gaseous oxygen, introduced via line 17a, will be readily dissolved in the feed stream ahead of first reactor 30 as shown. The mixed feed stream with dissolved oxygen is then fed simultaneously and continuously into plug flow reactor 30 with paraxylene being fed via line 31, and the reaction is initiated. The paraxylene may optionally be pre-mixed with acetic acid solvent and the mixture fed via line 31. Optionally, as described above, a portion of paraxylene feed 31 may bypass reactor 30 and be fed directly to second reactor 20. In cases where a portion of paraxylene feed 31 is fed directly to second reactor 20, the resulting solvent:paraxylene mass ratio in the reaction medium in the first reactor will adjust upwardly in response to that portion of the paraxylene feed which bypasses the first reactor, and resulting mass ratio may, therefore, reach a value in the range of from 80:1 up to values in the range of 100:1 and even higher

Molecular oxygen is dissolved in the mixed feed stream using any convenient in-line mixing device 33 to achieve a concentration of dissolved oxygen in the mixed feed stream of up to 3.0% w/w . Mixing device 33 could be an in-line nozzle arranged to discharge oxygen directly into the feed stream. In-line static mixers (not shown) can also be positioned upstream of first reactor 30 to facilitate mixing.

It is also possible according to the invention to stage the introduction of oxygen, i.e., to introduce the oxygen at a plurality of locations along the length of first reaction zone 30. By staging oxygen injection, the maximum local oxygen concentration is reduced, and this, in turn, permits a reduction in reactor operating pressure.

In practice, feed stream 10 is fed into plug flow reactor 30 simultaneously and continuously with paraxylene to thereby form a reaction medium in which the resulting solvent:paraxylene ratio is at least about 10:1, although the solvent:paraxylene ratio can be as high as 25:1 or even higher. In a preferred embodiment the solvent:paraxylene ratio is in the range of 10-20:1.

Residence time of the reaction medium within plug flow reaction zone 30 is relatively short, i.e., less than 6 minutes.

The reactor 30 shown in Fig. 2 is a shell and tube design. The reaction medium flows through the tubes, while a coolant, e.g., pressurized water (PW), is introduced into the shell side where it boils and is removed as steam (S). A small water purge (boiler blowdown, BB) is taken to control impurity/residue build-up in the water system.

The temperature of the reaction medium as it exits first reactor 30 is maintained below about 210°C by controlling the pressure of the produced steam, and hence its temperature. Controlling the process parameters as described according to the invention makes it possible to limit the uptake of oxygen within the reaction medium in the first reaction zone to a value which is less than 50% of the oxygen required for full conversion of the paraxylene to TA. Thus, paraxylene is converted in first reactor 30 primarily to TA intermediates, such as p-tolualdehyde, p-toluic acid and 4-CBA. Under the described process conditions, with effective temperature control of the reaction medium as it exits first reactor 30, the first reactor will not produce any solid TA.

Although a shell and tube reactor design is shown in Fig. 2, reactor 30 can be any suitable reactor design with optional heat removal and optional multiple oxygen injection. For example, the reactor can have multiple tube passes, with oxygen injection into the reaction medium upstream of each tube pass. Alternatively, a back-mixed reactor, such as, for example, a pumped circulating loop reactor, can be employed, with oxygen injection into the loop and heat removal from the loop as illustrated in Fig. 3. The reactor can also comprise a series of cooled or uncooled vessels, with optional oxygen injection upstream of each vessel.

The reaction medium exiting plug-flow first reactor 30 is fed via line 19 as described above in connection with the process embodiment shown in Fig. 1, to a second reactor, i.e., oxidation zone, 20, which, as shown, can be a conventional, continuously stirred tank reactor. Simultaneously, the pressure of the reaction medium is reduced to a value in the range of from 1,200 kPa to 2,000 kPa. Pressure reduction can be conveniently accomplished by passing the reaction medium through one or a plurality of pressure letdown valves or nozzles 21 positioned about the periphery of reactor 20 whereby the reaction medium is dispersed rapidly by injection into an agitator impeller region below the liquid line of the reactor. Process conditions within reactor 20, i.e., temperature, pressure, catalyst concentration, and residence time, are within conventional ranges, although oxygen uptake is reduced for reduced oxidation intensity.

A fresh supply of air or oxygen-containing gas, line 22a, is introduced and rapidly dispersed into the reaction medium in second reactor 20 by any convenient means.

TA will precipitate to form a slurry within reactor 20, and it can be recovered from the reactor system via line 23 using conventional methods. Overhead vapor from reactor 20, which will necessarily contain some acetic acid and water, is condensed via condenser 24, and most of the condensate is returned, i.e., recycled, via line 12 for feed stream make-up to first reactor 30. A portion of the acetic acid and water condensate stream (so-called water draw off) is diverted to a solvent dehydration system to remove the water of reaction. Optionally, but not shown, a portion of the condensate may be returned to reactor 20, to the reactor headspace, via a reflux slinger, and/or to the reaction zone, via a separate feed line or by mixing with the existing feed stream, line 19.

## Claims

1. A process for producing a carboxylic acid or its ester by catalytic liquid phase oxidation of a corresponding precursor in a solvent selected from an aliphatic carboxylic acid or a non-aliphatic organic acid, said solvent optionally including water, which comprises:
(a) forming a feed stream comprising solvent and oxidation catalyst at an elevated pressure of at least about 2,000 kPa;
(b) continuously and simultaneously feeding (1) the feed stream, (2) said precursor and (3) a supply of oxygen to a first reaction zone to form a reaction medium in which the solvent:precursor ratio is in the range of from at least 10:1;
(c) limiting the uptake of oxygen within the reaction medium in said first reaction zone to a value which is less than 50% of the oxygen required for full conversion of said precursor to its corresponding carboxylic acid or its ester; and
(d) feeding the reaction medium to a second reaction zone while simultaneously reducing the pressure of the reaction medium to a value in the range of from 1,200 kPa to 2,000 kPa.

2. The process of Claim 1 in which feeding the reaction medium to a second reaction zone while simultaneously reducing the pressure of the reaction medium to a value in the range of from 1,200 kPa to 2,000 kPa further comprises: (a) within the second reaction zone vaporizing a portion of the solvent present in the reaction medium; (b) removing the vapor from the reactor overhead; (c) condensing the vapor; and (d) recycling some or all of the condensate to the feed stream.

3. The process of Claim 1 which includes the additional step of recovering the resulting carboxylic acid or its ester from the second reaction zone.

4. The process of Claim 2 which includes the additional step of recovering the resulting carboxylic acid or its ester from the second reaction zone.

5. The process of Claim 1 in which the carboxylic acid is terephthalic acid, the precursor is paraxylene, and the solvent is acetic acid.

6. A process for producing a carboxylic acid or its ester by catalytic liquid phase oxidation of a corresponding precursor in a solvent selected from an aliphatic carboxylic acid or a non-aliphatic organic acid, said solvent optionally including water, which comprises:
(a) forming a feed stream comprising solvent and oxidation catalyst at an elevated pressure in the range of from 2,000 up to 20,000 kPa;
(b) oxygenating the feed stream;
(c) continuously and simultaneously feeding (1) the oxygenated feed stream and (2) said precursor to a first reaction zone to form a reaction medium in which the solvent:precursor ratio is in the range of from 10-30:1 and reaction products are maintained in solution as they are formed;
(d) limiting the uptake of oxygen within the reaction medium in said first reaction zone to a value which is less than 50% of the oxygen required for full conversion of said precursor to its corresponding carboxylic acid or its ester; and
(e) feeding the reaction medium to a second reaction zone while simultaneously reducing the pressure of the reaction medium to a value in the range of from 1,200 kPa to 2,000 kPa.

7. The process of Claim 6 wherein said first reaction zone is a plug flow reactor or a back-mixed reactor.

8. The process of Claim 6 in which feeding the reaction medium to a second reaction zone while simultaneously reducing the pressure of the reaction medium to a value in the range of from 1,200 kPa to 2,000 kPa further comprises: (a) within the second reaction zone vaporizing a portion of the solvent present in the reaction medium; (b) removing the vapor from the reactor overhead; (c) condensing the vapor; and (d) recycling some or all of the condensate to the feed stream.

9. The process of Claim 7 in which feeding the reaction medium to a second reaction zone while simultaneously reducing the pressure of the reaction medium to a value in the range of from 1,200 kPa to 2,000 kPa further comprises: (a) within the second reaction zone vaporizing a portion of the solvent present in the reaction medium; (b) removing the vapor from the reactor overhead; (c) condensing the vapor; and (d) recycling some or all of the condensate to the feed stream.

10. The process of Claim 6 which includes the additional step of recovering the resulting carboxylic acid or its ester from the second reaction zone.

11. The process of Claim 7 which includes the additional step of recovering the resulting carboxylic acid or its ester from the second reaction zone.

12. The process of Claim 8 which includes the additional step of recovering the resulting carboxylic acid or its ester from the second reaction zone.

13. The process of Claim 9 which includes the additional step of recovering the resulting carboxylic acid or its ester from the second reaction zone.

14. The process of Claim 6 in which the carboxylic acid is terephthalic acid, the precursor is paraxylene, and the solvent is acetic acid.

15. A process for catalytic liquid phase oxidation of paraxylene in a solvent comprising acetic acid which comprises:
(a) forming a feed stream comprising solvent and oxidation catalyst at an elevated pressure in the range of from 2,000 up to 20,000 kPa;
(b) oxygenating the feed stream;
(c) continuously and simultaneously feeding (1) the oxygenated feed stream and (2) said paraxylene to a first plug-flow reaction zone to form a reaction medium in which the acetic acid:paraxylene ratio is in the range of from 10-25:1 and reaction products are maintained in solution as they are formed;
(d) limiting the uptake of oxygen within the reaction medium in said first reaction zone to a value which is less than 50% of the oxygen required for full conversion of said paraxylene to terephthalic acid;
(e) feeding the reaction medium to a second reaction zone while simultaneously reducing the pressure of the reaction medium to a value in the range of from 1,200 kPa to 2,000 kPa whereby a portion of the solvent present in the reaction medium is vaporized; and
(f) continuously removing and condensing the vapor from the reactor overhead and recycling some or all of the condensate to the feed stream while recovering the resulting terephthalic acid from the second reaction zone.

16. The process of Claim 1, Claim 6 or Claim 15 which includes the additional step of diverting a portion of the paraxylene feed from said first reaction zone to said second reaction zone whereby said acetic acid:paraxylene ratio in said first reacton zone increases to a value in excess of 25:1.

## Patentansprüche

1. Verfahren zur Herstellung einer Carbonsäure oder eines Esters derselben durch eine katalytische Flüssigphasenoxidation eines entsprechenden Vorläuferproduktes in einem Lösungsmittel, das ausgewählt wird unter einer aliphatischen Carbonsäure oder einer nicht aliphatischen, organischen Säure, wobei das besagte Lösungsmittel wahlweise Wasser enthält, welches umfasst:
a) ein Bilden eines Zufuhrstromes, der ein Lösungsmittel und einen Oxydationskatalysator bei einem erhöhten Druck von mindestens etwa 2.000 kPa enthält;
b) ein kontinuierliches und gleichzeitiges Zuführen (1) des Zufuhrstromes, (2) des besagten Vorläuferproduktes und (3) einer Sauerstoffversorgung zu einer ersten Reaktionszone, um ein Reaktionsmedium zu bilden, in welchem das Verhältnis Lösungsmittel: Vorläuferprodukt in dem Bereich von mindestens 10:1 liegt;
c) ein Begrenzen der Aufnahme des Sauerstoffes innerhalb des Reaktionsmediums in der besagten ersten Reaktionszone auf einen Wert, der geringer ist als 50 % des Sauerstoffs, der erfordert ist für eine vollständige Umwandlung des besagten Vorläuferproduktes zu seiner entsprechenden Carbonsäure oder zu seinem Ester; und
d) ein Zuführen des Reaktionsmediums zu einer zweiten Reaktionszone, während gleichzeitig der Druck des Reaktionsmediums auf einen Wert in dem Bereich von 1.200 kPa bis zu 2.000 kPa vermindert wird.

2. Verfahren gemäß Anspruch 1, bei welchem das Zuführen des Reaktionsmediums zu einer zweiten Reaktionszone, bei einem gleichzeitigen Vermindern des Druckes des Reaktionsmediums auf einen Wert in dem Bereich von 1.200 kPa bis zu 2.000 kPa, weiterhin umfasst: (a) innerhalb der zweiten Reaktionszone ein Verdampfen eines Teiles des in dem Reaktionsmedium vorhandenen Lösungsmittels; (b) ein Entfernen des Dampfes aus dem Reaktoroberteil; (c) ein Kondensieren des Dampfes; und (d) ein Zurückführen eines Teiles oder der gesamten Menge des Kondensats zu dem Zufuhrstrom.

3. Verfahren gemäß Anspruch 1, welches den zusätzlichen Schritt des Zurückgewinnens der resultierenden Carbonsäure oder des Esters derselben aus der zweiten Reaktionszone umfasst.

4. Verfahren gemäß Anspruch 2, welches den zusätzlichen Schritt des Zurückgewinnens der resultierenden Carbonsäure oder des Esters derselben aus der zweiten Reaktionszone umfasst.

5. Verfahren gemäß Anspruch 1, bei welchem die Carbonsäure Terephthalsäure ist, das Vorläuferprodukt Paraxylen ist und das Lösungsmittel Essigsäure ist.

6. Verfahren zur Herstellung einer Carbonsäure oder des Esters derselben durch eine katalytische Flüssigphasenoxidation eines entsprechenden Vorläuferproduktes in einem Lösungsmittel, das ausgewählt wird unter einer aliphatischen Carbonsäure oder einer nicht aliphatischen, organischen Säure, wobei das besagte Lösungsmittel wahlweise Wasser enthält, welches umfasst:
a) ein Bilden eines Zufuhrstromes, der ein Lösungsmittel und einen Oxydationskatalysator bei einem erhöhten Druck in dem Bereich von 2.000 bis zu 20.000 kPa enthält;
b) ein Oxidieren des Zufuhrstromes;
c) ein kontinuierliches und gleichzeitiges Zuführen (1) des oxidierten Zufuhrstromes und (2) des besagten Vorläuferproduktes zu einer ersten Reaktionszone, um ein Reaktionsmedium zu bilden, in welchem das Verhältnis Lösungsmittel: Vorläuferprodukt in dem Bereich von 10 bis 30:1 liegt und in welchem die Reaktionsprodukte in Lösung gehalten werden, während sie gebildet werden;
d) ein Begrenzen der Aufnahme des Sauerstoffes innerhalb des Reaktionsmediums in der besagten ersten Reaktionszone auf einen Wert, der geringer ist als 50 % des Sauerstoffs, der erfordert ist für eine vollständige Umwandlung des besagten Vorläuferproduktes zu seiner entsprechenden Carbonsäure oder zu seinem Ester; und
e) ein Zuführen des Reaktionsmediums zu einer zweiten Reaktionszone, während gleichzeitig der Druck in dem Reaktionsmedium auf einen Wert in dem Bereich von 1.200 kPa bis zu 2.000 kPa vermindert wird.

7. Verfahren gemäß Anspruch 6, bei welchem die besagte erste Reaktionszone ein Pfropfenströmungs- oder ein Rückvermischungs-Reaktor ist.

8. Verfahren gemäß Anspruch 6, bei welchem das Zuführen des Reaktionsmediums zu einer zweiten Reaktionszone, bei einem gleichzeitigen Vermindern des Druckes des Reaktionsmediums auf einen Wert in dem Bereich von 1.200 kPa bis zu 2.000 kPa, weiterhin umfasst: (a) innerhalb der zweiten Reaktionszone ein Verdampfen eines Teiles des in dem Reaktionsmedium vorhandenen Lösungsmittels; (b) ein Entfernen des Dampfes aus dem Reaktoroberteil; (c) ein Kondensieren des Dampfes; und (d) ein Zurückführen eines Teiles oder der gesamten Menge des Kondensats zu dem Zufuhrstrom.

9. Verfahren gemäß Anspruch 7, bei welchem das Zuführen des Reaktionsmediums zu einer zweiten Reaktionszone, bei einem gleichzeitigen Vermindern des Druckes des Reaktionsmediums auf einen Wert in dem Bereich von 1.200 kPa bis zu 2.000 kPa, weiterhin umfasst: (a) innerhalb der zweiten Reaktionszone ein Verdampfen eines Teiles des in dem Reaktionsmedium vorhandenen Lösungsmittels; (b) ein Entfernen des Dampfes aus dem Reaktoroberteil; (c) ein Kondensieren des Dampfes; und (d) ein Zurückführen eines Teiles oder der gesamten Menge des Kondensats zu dem Zufuhrstrom.

10. Verfahren gemäß Anspruch 6, welches den zusätzlichen Schritt des Zurückgewinnens der resultierenden Carbonsäure oder des Esters derselben aus der zweiten Reaktionszone umfasst.

11. Verfahren gemäß Anspruch 7, welches den zusätzlichen Schritt des Zurückgewinnens der resultierenden Carbonsäure oder des Esters derselben aus der zweiten Reaktionszone umfasst.

12. Verfahren gemäß Anspruch 8, welches den zusätzlichen Schritt des Zurückgewinnens der resultierenden Carbonsäure oder des Esters derselben aus der zweiten Reaktionszone umfasst.

13. Verfahren gemäß Anspruch 9, welches den zusätzlichen Schritt des Zurückgewinnens der resultierenden Carbonsäure oder des Esters derselben aus der zweiten Reaktionszone umfasst.

14. Verfahren gemäß Anspruch 6, bei welchem die Carbonsäure Terephthalsäure ist, das Vorläuferprodukt Paraxylen ist und Lösungsmittel Essigsäure ist.

15. Verfahren zur katalytischen Flüssigphasenoxidation von Paraxylen in einem Essigsäure enthaltenden Lösungsmittel, welches umfasst:
a) ein Bilden eines Zufuhrstromes, welcher ein Lösungsmittel und einen Oxydationskatalysator bei einem erhöhten Druck in dem Bereich von 2.000 bis zu 20.000 kPa enthält;
b) ein Oxidieren des Zufuhrstromes;
c) ein kontinuierliches und gleichzeitiges Zuführen (1) des oxidierten Zufuhrstromes und (2) des besagten Paraxylens zu einer ersten Reaktionszone in dem Idealströmungsreaktor, um ein Reaktionsmedium zu bilden, in welchem das Verhältnis Essigsäure: Paraxylen in dem Bereich von 10 bis 25:1 liegt und in welchem die Reaktionsprodukte in dem Maße in Lösung gehalten werden, wie sie gebildet werden;
d) ein Begrenzen der Aufnahme des Sauerstoffes innerhalb des Reaktionsmediums in der besagten ersten Reaktionszone auf einen Wert, der geringer ist als 50% des Sauerstoffs, der erfordert ist für eine vollständige Umwandlung des besagten Paraxylens zu Terephthalsäure;
e) ein Zuführen des Reaktionsmediums zu einer zweiten Reaktionszone, während gleichzeitig der Druck in dem Reaktionsmedium auf einen Wert in dem Bereich von 1.200 kPa bis zu 2.000 kPa vermindert wird, wodurch ein Teil des in dem Reaktionsmedium vorhandenen Lösungsmittels verdunstet; und
f) ein kontinuierliches Entfernen und Kondensieren des Dampfes aus dem Reaktoroberteil und ein Zurückführen eines Teiles oder der gesamten Menge des Kondensats zu dem Zufuhrstrom, während die resultierende Terephthalsäure aus der zweiten Reaktionszone zurückgeführt wird

16. Verfahren gemäß Anspruch 1, Anspruch 6 oder Anspruch 15, welches den zusätzlichen Schritt des Umleitens eines Teiles der Paraxylenzufuhr aus der besagten ersten Reaktionszone zu der besagten zweiten Reaktionszone umfasst, wodurch das besagte Verhältnis Essigsäure: Paraxylen in der besagten ersten Reaktionszone auf einen Wert von mehr als 25:1 anwächst.

## Revendications

1. Procédé de production d'un acide carboxylique ou de son ester par oxydation catalytique en phase liquide d'un précurseur correspondant dans un solvant choisi parmi un acide carboxylique aliphatique ou un acide organique non aliphatique, ledit solvant incluant éventuellement de l'eau, qui comprend:
(a) la formation d'un courant d'alimentation comprenant le solvant et le catalyseur d'oxydation à une pression élevée d'au moins environ 2 000 kPa;
(b) l'alimentation continue et simultanée (1) du courant d'alimentation, (2) dudit précurseur et (3) d'une source d'alimentation en oxygène à une première zone réactionnelle pour former un milieu réactionnel dans lequel le rapport solvant: précurseur est situé dans la plage d'au moins 10: 1;
(c) la limitation du prélèvement en oxygène à l'intérieur du milieu réactionnel dans ladite première zone réactionnelle jusqu'à une valeur qui est inférieure à 50 % de l'oxygène requis pour la conversion complète dudit précurseur en son acide carboxylique ou son ester correspondant; et
(d) l'alimentation du milieu réactionnel à une seconde zone réactionnelle tout en réduisant simultanément la pression du milieu réactionnel à une valeur située dans la plage de 1 200 kPa à 2 000 kPa.

2. Procédé selon la revendication 1, dans lequel l'alimentation du milieu réactionnel à une seconde zone réactionnelle tout en réduisant simultanément la pression du milieu réactionnel à une valeur située dans la plage de 1 200 kPa à 2 000 kPa comprend en outre: (a) à l'intérieur de la seconde zone réactionnelle la vaporisation d'une partie du solvant présent dans le milieu réactionnel; (b) l'élimination de la vapeur de la zone supérieure du réacteur; (c) la condensation de la vapeur; et (d) le recyclage d'une partie ou de tout le condensat vers le courant d'alimentation.

3. Procédé selon la revendication 1, qui inclut l'étape supplémentaire de récupération de l'acide carboxylique ou de son ester résultant de la seconde zone réactionnelle.

4. Procédé selon la revendication 2, qui inclut l'étape supplémentaire de récupération de l'acide carboxylique ou de son ester résultant de la seconde zone réactionnelle.

5. Procédé selon la revendication 1, dans lequel l'acide carboxylique est l'acide téréphtalique, le précurseur est le paraxylène, et le solvant est l'acide acétique.

6. Procédé de production d'un acide carboxylique ou de son ester par oxydation catalytique en phase liquide d'un précurseur correspondant dans un solvant choisi parmi un acide carboxylique aliphatique ou un acide organique non aliphatique, ledit solvant incluant éventuellement de l'eau, qui comprend:
(a) la formation d'un courant d'alimentation comprenant le solvant et le catalyseur d'oxydation à une pression élevée située dans la plage de 2 000 kPa à 20 000 kPa;
(b) l'oxygénation du courant d'alimentation;
(c) l'alimentation continue et simultanée (1) du courant d'alimentation oxygéné et (2) dudit précurseur jusqu'à une première zone réactionnelle pour former un milieu réactionnel dans lequel le rapport solvant: précurseur se situe dans la plage de 10 à 30: 1 et les produits réactionnels sont maintenus en solution lorsqu'ils sont formés;
(d) la limitation du prélèvement en oxygène à l'intérieur du milieu réactionnel dans ladite première zone réactionnelle jusqu'à une valeur qui est inférieure à 50 % de l'oxygène requis pour la conversion complète dudit précurseur en son acide carboxylique ou son ester correspondant; et
(e) l'alimentation du milieu réactionnel jusqu'à une seconde zone réactionnelle tout en réduisant simultanément la pression du milieu réactionnel à une valeur située dans la plage de 1 200 kPa à 2 000 kPa.

7. Procédé selon la revendication 6 dans lequel ladite première zone réactionnelle est un réacteur à flux piston ou un réacteur à contre-courant.

8. Procédé selon la revendication 6, dans lequel l'alimentation du milieu réactionnel jusqu'à une seconde zone réactionnelle tout en réduisant simultanément la pression du milieu réactionnel à une valeur située dans la plage de 1 200 kPa à 2 000 kPa comprend en outre: (a) à l'intérieur de la seconde zone réactionnelle la vaporisation d'une partie du solvant présent dans le milieu réactionnel; (b) l'élimination de la vapeur de la zone supérieure du réacteur; (c) la condensation de la vapeur; et (d) le recyclage d'une partie ou de tout le condensat vers le courant d'alimentation.

9. Procédé selon la revendication 7, dans lequel l'alimentation du milieu réactionnel jusqu'à une seconde zone réactionnelle tout en réduisant simultanément la pression du milieu réactionnel jusqu'à une valeur située dans la plage de 1 200 kPa à 2 000 kPa comprend en outre (a) à l'intérieur de la seconde zone réactionnelle la vaporisation d'une partie du solvant présent dans le milieu réactionnel; (b) l'élimination de la vapeur de la partie supérieure du réacteur; (c) la condensation de la vapeur; et (d) le recyclage d'une partie ou de tout le condensat jusqu'au courant d'alimentation.

10. Procédé selon la revendication 6 qui inclut l'étape supplémentaire de récupération de l'acide carboxylique ou de son ester résultant à partir de la seconde zone réactionnelle.

11. Procédé selon la revendication 7, qui inclut l'étape supplémentaire de récupération de l'acide carboxylique ou de son ester résultant à partir de la seconde zone réactionnelle.

12. Procédé selon la revendication 8, qui inclut l'étape supplémentaire de récupération de l'acide carboxylique ou de son ester résultant à partir de la seconde zone réactionnelle.

13. Procédé selon la revendication 9, qui inclut l'étape supplémentaire de récupération de l'acide carboxylique ou de son ester résultant à partir de la seconde zone réactionnelle.

14. Procédé selon la revendication 6, dans lequel l'acide carboxylique est l'acide téréphtalique, le précurseur est le paraxylène, et le solvant est l'acide acétique.

15. Procédé pour l'oxydation catalytique en phase liquide de paraxylène dans un solvant comprenant de l'acide acétique qui comprend:
(a) la formation d'un courant d'alimentation comprenant le solvant et le catalyseur d'oxydation à une pression élevée située dans la plage de 2 000 kPa jusqu'à 20 000 kPa;
(b) l'oxygénation du courant d'alimentation;
(c) l'alimentation de manière continue et simultanée (1) du courant d'alimentation oxygéné et (2) dudit paraxylène jusqu'à une première zone réactionnelle à flux piston pour former un milieu réactionnel dans lequel le rapport acide acétique: paraxylène se situe dans la plage de 10 à 25:1 et les produits réactionnels sont maintenus en solution lorsqu'ils sont formés.
(d) la limitation du prélèvement en oxygène à l'intérieur du milieu réactionnel dans ladite première zone réactionnelle jusqu'à une valeur qui est inférieure à 50 % de l'oxygène requis pour la conversion entière dudit paraxylène en acide téréphtalique;
(e) l'alimentation du milieu réactionnel jusqu'à une seconde zone réactionnelle tout en réduisant simultanément la pression du milieu réactionnel à une valeur située dans la plage de 1 200 kPa à 2 000 kPa moyennant quoi une partie du solvant présent dans le milieu réactionnel est vaporisée; et
(f) l'élimination et la condensation de manière continue de la vapeur de la zone supérieure du réacteur et le recyclage d'une partie ou de tout le condensat jusqu'au courant d'alimentation tout en récupérant l'acide téréphtalique résultant de la seconde zone réactionnelle.

16. Procédé selon la revendication 1, revendication 6 ou revendication 15, qui inclut l'étape supplémentaire de détournement d'une partie de l'alimentation en paraxylène de ladite première zone réactionnelle jusqu'à ladite seconde zone réactionnelle moyennant quoi ledit rapport acide acétique: paraxylène dans ladite première zone réactionnelle augmente jusqu'à une valeur en excès de 25:1.
